# EUROPÄISCHE PATENTANMELDUNG

(11) **EP 1 905 403 A1**
(43) Veröffentlichungstag der Anmeldung: **02.04.2008**
(21) Anmeldenummer: 06020096.1
(22) Anmeldetag: 26.09.2006
(51) Int. Cl.: A61F 15/00

(54) **Spendersystem**

(71) Anmelder: Kretzer, Thorsten, 51381 Leverkusen (DE); Müller, Klaus, 51465 Bergisch Gladbach (DE)
(72) Erfinder: Kretzer, Thorsten, 51381 Leverkusen (DE); Müller, Klaus, 51465 Bergisch Gladbach (DE)
(74) Vertreter: Paul, Dieter-Alfred

(57) **Zusammenfassung**

Die Erfindung betrifft ein Spendersystem mit einem Gehäuse (2) und einer darin ausgebildeten Entnahmeöffnung (3), das eine Vielzahl von saugfähigen Pads (1) für die Aufnahme einer kleinen Menge von Urin enthält, wobei die einzelnen Pads (1) aus der Entnahmeöffnung (3) des Spendersystems so herausnehmbar sind, dass die im Spendersystem verbleibenden Pads (1) vor Verunreinigungen geschützt sind, und wobei die Pads (1) mindestens eine flüssigkeitsaufnehmende (5) und eine flüssigkeitsdichte (6) Schicht aufweisen.

## Beschreibung

Die Erfindung betrifft ein Spendersystem, das eine Vielzahl von saugfähigen Pads für die Aufnahme einer kleinen Menge von Urin enthält.

Im Stand der Technik sind verschiedene absorbierende Artikel, unter anderem auch für Urin, beschrieben. So ist beispielsweise in der DE 699 15 512 T2 ein derartiger Artikel aus einem Mehrschichtmaterial offenbart, der einen Absorptionskörper zwischen einer flüssigkeitsundurchlässigen Außenschicht und einer flüssigkeitsdurchlässigen Innenschicht aufweist. Die im Stand der Technik beschriebenen Absorptionsprodukte werden u.a. für Windeln, Hygienebinden, Inkontinenzschutz, Wundverband und ähnliches verwendet.

Wenn Männer urinieren, bleibt am Penis eine geringe Menge Urin, d.h. wenige Tropfen, zurück. Dieser Urin kann bei der Benutzung einer normalen Toilette mit Hilfe von Toilettenpapier entfernt werden. Wenn der Mann jedoch ein Urinal verwendet, besteht diese Möglichkeit nicht.

Es ist daher Aufgabe der vorliegenden Erfindung, ein Spendersystem bereit zu stellen, das saugfähige Pads enthält, die eine kleine Menge Urin absorbieren können, wobei das Spendersystem hygienischen Anforderungen genügen soll.

Diese Aufgaben werden erfindungsgemäß durch ein Spendersystem mit einem Gehäuse und einer darin ausgebildeten Entnahmeöffnung, das eine Vielzahl von saugfähigen Pads für die Aufnahme einer kleinen Menge von Urin enthält, wobei die einzelnen Pads aus der Entnahmeöffnung des Spendersystems so herausnehmbar sind, dass die im Spendersystem verbleibenden Pads vor Verunreinigungen geschützt sind, und wobei die Pads mindestens eine flüssigkeitsaufnehmende und eine flüssigkeitsdichte Schicht aufweisen, gelöst.

Grundgedanke der Erfindung ist es also, ein Spendersystem mit einem Gehäuse und einer darin ausgebildeten Entnahmeöffnung bereit zu stellen, in dem eine Vielzahl von saugfähigen Pads enthalten ist. Die Pads weisen mindestens eine flüssigkeitsaufnehmende und eine flüssigkeitsdichte Schicht auf, so dass sie in der Lage sind, eine kleine Menge von Urin absorbierend aufzunehmen, ohne dass das Pad vollständig von dem Urin durchtränkt wird. Das Spendersystem ist weiterhin so ausgelegt, dass von einem Benutzer die Pads einzeln aus der Entnahmeöffnung heraus genommen werden können, ohne dass die in dem Spendersystem verbleibenden Pads durch den Benutzer verunreinigt werden.

Vorteil des erfindungsgemäßen Spendersystems ist, dass ein Benutzer, der beispielsweise an einem Urinal uriniert hat, aus diesem ein saugfähiges Pad entnehmen kann, ohne dass die in dem Spendersystem verbleibenden Pads verschmutzt werden, wobei er mit dem entnommenen Pad die an seinem Penis verbliebene Menge Urin abtupfen kann. Dabei wird der Urin von der flüssigkeitsaufnehmenden Schicht absorbiert und die flüssigkeitsdichte Schicht verhindert, dass der Benutzer mit dem Urin in Kontakt kommt.

Gemäß einer Ausführungsform der Erfindung ist vorgesehen, dass das Spendersystem eine Halterung zum Anbringen an einer Wand, insbesondere neben einem Urinal, aufweist. Vorteil ist hierbei dabei, dass das Spendersystem leicht neben dem Urinal angebracht werden kann, so dass ein Benutzer direkten Zugriff hat.

Es ist ebenfalls möglich, das Spendersystem transportabel auszubilden. In diesem Fall ist es möglich, dass ein Mann das System beispielsweise auf einem Wanderausflug mit sich führt und verwendet, wenn er im Freien urinieren muss.

Gemäß einer weiteren Ausführungsform ist vorgesehen, dass das Spendersystem eine Aufnahme-Einrichtung für benutzte Pads aufweist. Diese kann außerdem einen geruchsdichten Verschluss besitzen. Hierdurch kann sichergestellt werden, dass die benutzten Pads einer ordnungsgemäßen Entsorgung zugeführt werden.

Die Pads können trennbar, insbesondere über eine Perforation, miteinander verbunden sein. Dies erleichtert die Entnahme der Pads aus dem Spendersystem sowie das Nachfüllen.

Es ist ebenfalls möglich, dass das Spendersystem eine Schneideeinrichtung zum Trennen der verbundenen Pads aufweist. Vorteilhaft ist hierbei, dass die Pads leicht voneinander getrennt werden können.

Die Pads können in dem Spendersystem in Rollenform enthalten sein. Hierdurch wird das Nachfüllen des Spendersystems vereinfacht.

Es ist ebenfalls möglich, dass in dem Spendersystem übereinander geschichtete Pads enthalten sind, wobei gemäß einer weiteren Ausführungsform der Erfindung vorgesehen ist, dass das Spendersystem eine Feder aufweist, die die übereinander geschichteten Pads in Richtung der Entnahmeöffnung drückt. Alternativ kann ein Gewicht vorgesehen werden, das ebenfalls die Pads in diese Richtung drückt. Auf diese Weise kann sichergestellt werden, dass alle in dem Spendersystem enthaltenen Pads zu der Entnahmeöffnung gelangen und entnommen werden können.

Um die Entnahme der Pads aus dem Spendersystem weiter zu erleichtern, können die Pads eine Anrauhung und/oder eine Haftbeschichtung aufweisen. Ebenfalls kann eine zusätzliche Lasche für die einfache Entnahme vorgesehen sein.

Gemäß einem weiteren Aspekt der Erfindung ist vorgesehen, dass die Pads mit einer entfernbaren hygienischen Schutzschicht versehen sind, die auf der flüssigkeitsaufnehmenden Schicht angebracht ist, wodurch diese vor einer Verunreinigung geschützt ist. Um einen vollständigen Schutz der gesamten Pads zu gewährleisten, können diese einzeln verpackt sein.

Die flüssigkeitsaufnehmende Schicht kann aus Papier, Vliesstoff, Watte oder Schwamm bestehen. Diese Stoffe sind vorteilhaft, da sie günstig sind und eine gute Absorptionsfähigkeit für Flüssigkeiten aufweisen.

Die flüssigkeitsdichte Schicht kann aus silikonisiertem Papier, Folie oder Laminat bestehen. Diese Verbindungen sind ebenfalls günstig und verhindern zuverlässig einen Durchtritt des von dem Pad absorbierten Urins.

Um eine sichere und stabile Verbindung der flüssigkeitsaufnehmenden und der flüssigkeitsdichten Schicht zu gewährleisten, ist gemäß einer weiteren Ausführungsform vorgesehen, die Schichten miteinander zu verkleben oder zu verschweißen.

Es ist ebenfalls möglich, dass die Pads ein Pflegemittel enthalten und/oder parfümiert sind.

Die Erfindung wird im folgenden anhand eines Ausführungsbeispiels mit Bezugnahme auf die Zeichnungen näher erläutert. In den Figuren zeigt:
- Figur 1: einen Querschnitt durch ein erstes erfindungsgemäßes Spendersystem, das eine Vielzahl von saugfähigen Pads enthält,
- Figur 2: eine Aufsicht auf ein erfindungsgemäßes Pad gemäß Figur 1, und
- Figur 3: einen Längsschnitt durch ein erfindungsgemäßes Pad gemäß Figur 1.

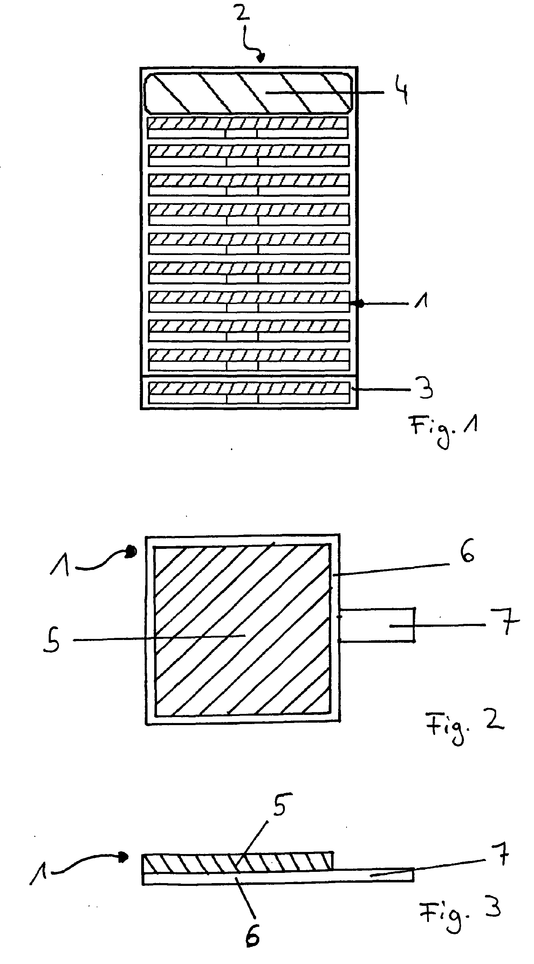

In der Figur 1 ist ein erfindungsgemäßes Spendersystem gezeigt, das eine Vielzahl von saugfähigen Pads 1 für die Aufnahme einer kleinen Menge Urin enthält. Das Spendersystem weist ein Gehäuse 2 und eine darin ausgebildete Entnahmeöffnung 3 auf. Im Inneren des Gehäuses 2 sind die Pads 1 übereinander geschichtet angeordnet. Auf dem in der Zeichnung obersten Pad 1 liegt ein Gewicht 4 auf, das die Pads 1 in Richtung der Entnahmeöffnung 3 drückt. Anstelle des Gewichtes 4 kann in dem Spendersystem auch eine Feder angeordnet sein, die die Pads nach unten in Richtung der Entnahmeöffnung 3 drückt.

Jeweils nur ein Pad 1 befindet sich in der Entnahmeöffnung 3, so dass bei dessen Entnahme durch einen Benutzer die übrigen Pads 1 innerhalb des Gehäuses 2 nicht von ihm berührt werden. Auf diese weise wird eine Verunreinigung der übrig bleibenden Pads 1 verhindert.

Die saugfähigen Pads 1 weisen eine flüssigkeitsaufnehmende Schicht 5 und eine flüssigkeitsdichte Schicht 6 auf. Die flüssigkeitsaufnehmende Schicht 5 besteht hier aus Vliesstoff und die flüssigkeitsdichte Schicht 6 aus silikonisiertem Papier. Beide Schichten 5, 6 sind miteinander verklebt. Zusätzlich ist das Pad 1 an der flüssigkeitsdichten Schicht 6 mit einer Lasche 7 versehen, die die Entnahme aus dem Spendersystem durch einen Benutzer erleichtert.

Die Pads 1 können mit einer Anrauhung und/oder einer Haftbeschichtung versehen werden, die die Entnahme aus dem Spendersystem erleichtert. Weiterhin kann zum Schutz der Pads 1 vor einer Verunreinigung vorgesehen sein, dass entweder die Flüssigkeit aufnehmende Schicht mit einer entfernbaren hygienischen Schutzschicht versehen ist oder dass die Pads 1 vollständig einzeln verpackt sind.

Die Pads 1 können ein Pflegemittel enthalten und/oder parfümiert sein.

Wenn ein Benutzer das erfindungsgemäße Spendersystem, das vorzugsweise neben einem Urinal an einer Wand angebracht sein kann, verwendet, entnimmt er aus der Entnahmeöffnung 3 das darin befindliche Pad 1, indem er die Lasche 7 des Pads 1 mit den Fingern fasst. Während dieses Herausnehmvorgangs werden die in dem Gehäuse 2 des Spendersystems zurück bleibenden Pads 1 nicht von dem Benutzer berührt und daher auch nicht kontaminiert. Nachdem das Pad 1 aus der Entnahmeöffnung 3 entnommen worden ist, werden die übrigen Pads 1 in dem Gehäuse 2 des Spendersystems von dem Gewicht 4 nach unten in Richtung der Entnahmeöffnung 3 gedrückt, so dass erneut ein Pad 1 in der Entnahmeöffnung 3 angeordnet wird.

Der Benutzer tupft mit dem entnommenen Pad 1 eine an seinem Penis verbliebene Restmenge Urin derart ab, dass er die flüssigkeitsaufnehmende Schicht 5 in Kontakt mit seinem Penis bringt, wobei die flüssigkeitsdichte Schicht 6 auf seiner Hand aufliegt. Dabei wird der Urin von der flüssigkeitsaufnehmenden Schicht 5 aufgesaugt und die flüssigkeitsdichte Schicht 6 verhindert den Kontakt des Urins mit der Hand des Benutzers.

Anschließend kann der Benutzer das verwendete Pad in eine nicht gezeigte Aufnahme-Einrichtung für die Entsorgung einwerfen. Dieses kann einen geruchsdichten Verschluss aufweisen.

Alternativ kann das Spendersystem transportabel ausgebildet sein. Es ist ebenfalls möglich, dass die Pads 1 trennbar, beispielsweise über eine Perforation, miteinander verbunden sind. In diesem Fall kann das Spendersystem eine Schneideeinrichtung zum Trennen der Pads 1 aufweisen. Außerdem können die Pads 1 in Rollenform in dem Spendersystem angeordnet sein.

## Patentansprüche

1. Spendersystem mit einem Gehäuse (2) und einer darin ausgebildeten Entnahmeöffnung (3), das eine Vielzahl von saugfähigen Pads (1) für die Aufnahme einer kleinen Menge von Urin enthält, wobei die einzelnen Pads (1) aus der Entnahmeöffnung (3) des Spendersystems so herausnehmbar sind, dass die im Spendersystem verbleibenden Pads (1) vor Verunreinigungen geschützt sind, und wobei die Pads (1) mindestens eine flüssigkeitsaufnehmende (5) und eine flüssigkeitsdichte (6) Schicht aufweisen.

2. Spendersystem nach Anspruch 1, **dadurch gekennzeichnet, dass** es eine Halterung zum Anbringen an einer Wand, insbesondere neben einem Urinal, aufweist.

3. Spendersystem nach Anspruch 1, **dadurch gekennzeichnet, dass** es transportabel ausgebildet ist.

4. Spendersystem nach einem der vorhergehenden Ansprüche, **dadurch gekennzeichnet, dass** es eine Aufnahme-Einrichtung für benutzte Pads (1) aufweist.

5. Spendersystem nach Anspruch 4, **dadurch gekennzeichnet, dass** die Aufnahme-Einrichtung einen geruchsdichten Verschluss aufweist.

6. Spendersystem nach einem der vorhergehenden Ansprüche, **dadurch gekennzeichnet, dass** die Pads (1) trennbar, insbesondere über eine Perforation, miteinander verbunden sind.

7. Spendersystem nach Anspruch 6, **dadurch gekennzeichnet, dass** eine Schneideinrichtung zum Trennen der verbundenen Pads (1) vorgesehen ist.

8. Spendersystem nach einem der Ansprüche 6 oder 7, **dadurch gekennzeichnet, dass** die Pads (1) in Rollenform in dem Gehäuse (2) enthalten sind.

9. Spendersystem nach einem der Ansprüche 1 bis 7, **dadurch gekennzeichnet, dass** es übereinander geschichtete Pads (1) enthält.

10. Spendersystem nach Anspruch 9, **dadurch gekennzeichnet, dass** es eine Feder aufweist, welche die Pads (1) in Richtung der Entnahmeöffnung drückt.

11. Spendersystem nach Anspruch 9, **dadurch gekennzeichnet, dass** es ein Gewicht (4) aufweist, das die Pads (1) in Richtung der Entnahmeöffnung drückt.

12. Spendersystem nach einem der vorhergehenden Ansprüche, **dadurch gekennzeichnet, dass** die Pads (1) eine Anrauung und/oder eine Haftbeschichtung für eine einfache Entnahme aufweisen.

13. Spendersystem nach einem der vorhergehenden Ansprüche, **dadurch gekennzeichnet, dass** die Pads (1) eine Lasche (7) für eine einfache Entnahme aufweisen.

14. Spendersystem nach einem der vorhergehenden Ansprüche, **dadurch gekennzeichnet, dass** die Pads (1) eine entfernbare hygienische Schutzschicht auf der flüssigkeitsaufnehmenden Schicht (5) aufweisen.

15. Spendersystem nach einem der vorhergehenden Ansprüche, **dadurch gekennzeichnet, dass** die Pads einzeln verpackt sind.

16. Spendersystem nach einem der vorhergehenden Ansprüche, **dadurch gekennzeichnet, dass** die flüssigkeitsaufnehmende Schicht (5) aus Papier, Vliesstoff, Watte oder Schwamm besteht.

17. Spendersystem nach einem der vorhergehenden Ansprüche, **dadurch gekennzeichnet, dass** die flüssigkeitsdichte Schicht (6) aus silikonisiertem Papier, Folie oder Laminat besteht.

18. Spendersystem nach einem der vorhergehenden Ansprüche, **dadurch gekennzeichnet, dass** die flüssigkeitsaufnehmende (5) und die flüssigkeitsdichte Schicht (6) miteinander verklebt oder verschweißt sind.

19. Spendersystem nach einem der vorhergehenden Ansprüche, **dadurch gekennzeichnet, dass** die Pads (1) ein Pflegemittel enthalten.

20. Spendersystem nach einem der vorhergehenden Ansprüche, **dadurch gekennzeichnet, dass** die Pads (1) parfümiert sind.
